# FASCICULE DE BREVET EUROPEEN

(11) **EP 3 375 433 B1**
(45) Date de publication et mention de la délivrance du brevet: **19.02.2020**
(21) Numéro de dépôt: 17305285.3
(22) Date de dépôt: 16.03.2017
(51) Int. Cl.: A61K 8/97, A61K 8/37, A61K 8/34, A61K 8/9789, A61Q 19/08

(54) **COMPOSITION COSMÉTIQUE COMPRENANT UN EXTRAIT DE MENTHE POIVRÉE**
KOSMETISCHE ZUSAMMENSETZUNG, DIE EIN PFEFFERMINZEXTRAKT ENTHÄLT
COSMETIC COMPOSITION COMPRISING A PEPPERMINT EXTRACT

(43) Date de publication de la demande: 19.09.2018
(73) Titulaire: Chanel Parfums Beauté, 92200 Neuilly-sur-Seine (FR)
(72) Inventeur: COCANDEAU, VINCENT, 93400 SAINT OUEN (FR); GILLET, MAEVA, 75012 PARIS (FR); HOLDERITH, Serge, 94300 VINCENNES (FR); LEGANGNEUX, DAVID, 78120 Rambouillet (FR); LERISSON, ANNIE, 95360 MONTMAGNY (FR); REY, AURELIEN, 75018 PARIS (FR); TORIBIO, ALIX, 93400 SAINT OUEN (FR); WEINBERG, Lionel, 94170 LE PERREUX-SUR-MARNE (FR)
(74) Mandataire: Plasseraud IP

(56) Documents cités:
- DE-A1-102005 026 357
- NAJAFIAN SHARAREH ET AL: "Polyphenolic contents and antioxidant activities of two medicinal plant species,Mentha piperitaandStevia rebaudiana, cultivated in Iran", COMPARATIVE CLINICAL PATHOLOGY, SPRINGER-VERLAG, LONDON, vol. 25, no. 4, 30 mars 2016 (2016-03-30), pages 743-747, XP036000414, ISSN: 1618-5641, DOI: 10.1007/S00580-016-2258-5 [extrait le 2016-03-30]
- ATIKORN PANYA ET AL: "An Investigation of the Versatile Antioxidant Mechanisms of Action of Rosmarinate Alkyl Esters in Oil-in-Water Emulsions", JOURNAL OF AGRICULTURAL AND FOOD CHEMISTRY, vol. 60, no. 10, 14 mars 2012 (2012-03-14) , pages 2692-2700, XP055226738, ISSN: 0021-8561, DOI: 10.1021/jf204848b

## Description

### Domaine de l'invention

La présente invention concerne une composition cosmétique ou dermatologique comprenant un extrait de menthe poivrée provenant d'au moins une espèce de Menthe (Mentha Piperita). En particulier, la présente invention concerne une composition cosmétique capable de réduire la présence de radicaux libres impliqués dans les phénomènes de vieillissement cellulaire de la peau. L'invention concerne également l'utilisation d'un extrait de menthe poivrée en tant qu'agent antioxydant dans une composition cosmétique ou dermatologique.

### Contexte de l'invention

La production de radicaux libres, est l'un des facteurs majeurs de l'accélération du vieillissement cutané. On sait aujourd'hui que 80% des radicaux libres externes proviennent de l'exposition UV, les 20% restants proviennent de la pollution et des variations climatiques. Il est donc important d'apporter à la peau des antioxydants, seuls nutriments capables de piéger ces molécules réactives qui s'attaquent aux protéines, aux acides gras et à l'ADN de nos cellules.

Le vieillissement, le photo-vieillissement de la peau et les altérations qui sont associées à la présence de radicaux libres peuvent se manifester de différentes manières, parmi lesquelles on peut citer:
- la perte de fermeté et d'élasticité dues à une perte tissulaire au niveau de l'épiderme et/ou du derme;
- la perte d'éclat due à la réduction de la microcirculation et à un ralentissement du renouvellement cellulaire au niveau de l'épiderme;
- l'apparition de taches pigmentaires associées à un dysfonctionnement de la synthèse de la mélanine (ou mélanogénèse) ;
- la sécheresse cutanée résultant d'une diminution de la fonction barrière de la couche cornée et à un ralentissement du renouvellement épidermique.

Il existe, de ce fait, un besoin de fournir un antioxydant susceptible de réduire la présence de radicaux libres et ainsi d'agir sur un ensemble de causes d'altérations de la peau dues au vieillissement et/ou à une modification des mécanismes physiologiques liés au vieillissement induit par les radicaux libres.

Il est de plus en plus fréquent d'utiliser des extraits d'origines naturelles dans le domaine dermo-cosmétique. La menthe poivrée est connue pour ses propriétés antioxydantes, calmantes, adoucissantes, analgésiques et anesthésiques. On connait l'acide rosmarinique, qui en est issu, comme l'une des molécules naturelles les plus antioxydantes qui existe. On sait également qu'il est possible de modifier les propriétés physicochimiques de certaines molécules par voie chimique ou biologique afin de moduler leurs efficacités et de cibler leurs actions au sein des cellules. Ainsi, en fonction de la polarité chacune des molécules obtenues aura une action donnée dans les différents compartiments cellulaires. Par exemple, on sait que le greffage d'une molécule de glycérol permet d'avoir une meilleure biodisponibilité dans les milieux aqueux.

Il existe donc une demande pour une composition cosmétique ou dermatologique présentant des propriétés antioxydantes permettant de lutter efficacement contre la présence de radicaux libres.

Toutefois, une telle composition obtenue à partir d'un extrait végétal et plus particulièrement de menthe poivrée nécessite un extrait aux propriétés antioxydantes élevées.

### Résumé de l'invention

La présente invention a été réalisée par rapport à l'art antérieur décrit ci-dessus, et le but de la présente invention est donc d'obtenir une composition cosmétique ou dermatologique qui, par ses capacités antioxydantes est capable de réduire très fortement la présence de radicaux libres impliqués dans les phénomènes de vieillissement cellulaire de la peau ainsi que l'utilisation d'une telle composition pour lutter contre la présence de radicaux libres.

Pour résoudre le problème, la présente invention fournit un extrait de menthe poivrée (I) obtenu par extraction des tiges de menthe poivrée avec un mélange de solvants hydro-alcooliques de préférence d'un mélange éthanol/eau et estérification avec du glycérol, ledit extrait de menthe poivrée (I) comprenant au moins un composé de formule (I) :

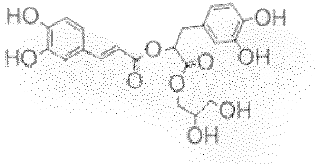

Ainsi, les présents inventeurs ont constaté que ledit extrait (I) permettait, par le biais de ses propriétés antioxydantes (démontrées à l'exemple 4 tableau 3), de lutter contre la présence de radicaux libres, ce qui leur a permis d'aboutir à la présente invention. Les propriétés antioxydantes de cet extrait permettent d'agir sur les symptômes dus au vieillissement, ou à des mécanismes physiologiques liés au vieillissement, ou à des troubles apparentés à ces mécanismes au niveau de l'épiderme et/ou du derme. Par composition cosmétique ou dermatologique dans la présente invention nous excluons les applications médicales.

Le produit d'une extraction hydro-alcoolique de tiges séchées de menthe poivrée et estérifié avec du glycérol est enrichi en composé de formule (I) et présente d'excellentes propriétés antioxydantes.

L'extrait(l) est préférentiellement obtenu d'au moins une espèce de Menthe poivrée (Mentha Piperita).

Le Genre Mentha appartient à la famille des *Lamiaceae.* Cette famille des *Lamiaceae* est une importante famille de plantes comprenant environ 6.000 espèces et près de 210 genres, largement répandues à travers le monde et dans tout type de milieux.

Les menthes sont des *Lamiaceaes* typiques (hormis la corolle des fleurs ne répondant pas au caractère bilabié) et appartiennent au genre Mentha. Ce genre comporte vingt-cinq espèces de vivaces aromatiques persistantes ou semi-persistantes d'Europe, d'Asie et d'Afrique. La plupart sont cultivées pour leur arôme, pour leur saveur ou leurs qualités ornementales. Leurs formes varient de rampantes à buissonnantes, et leur saveur peut être rafraîchissante à très forte.

L'espèce Mentha × piperita L. est un hybride créé en Angleterre, issu d'un croisement entre Mentha spicata et Mentha aquatica, elle a pour synonyme *Mentha x adspersa* Moench, *Mentha x atrata* Ehrh., *Mentha x balsamea* Willd., *Mentha x concinna* Pérard, *Mentha x eriantha* K.Koch, *Mentha x odora* Salisb., *Mentha x piperoides* Malinv., *Mentha x reverchonii* Briq., *Mentha nigricans* Mill., *Mentha odorata* Sole.
Elle est également connue sous les noms communs : peppermint, mint, peppermin, menthe poivrée, Pfefferminze.

La présente invention a également pour objet une composition cosmétique ou dermatologique, caractérisée en ce qu'elle comprend, dans un milieu physiologiquement acceptable, un extrait de menthe poivrée (I) tel que défini précédemment.

Avantageusement, la composition comprend également un extrait hydro-alcoolique de tiges de menthe poivrée (II) obtenu par extraction des tiges avec un mélange de solvants hydro-alcooliques, de préférence d'un mélange éthanol/eau, comprenant au moins un composé de formule (II) 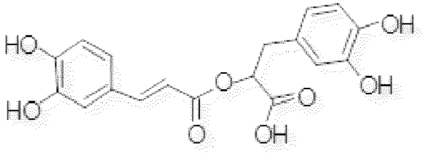 et un extrait de menthe poivrée (III) obtenu par extraction des tiges de menthe poivrée avec un mélange de solvants hydro-alcooliques, de préférence avec un mélange éthanol/eau et estérification avec de l'alcool octylique, ledit extrait de menthe poivrée (III) comprenant au moins un composé de formule (III)

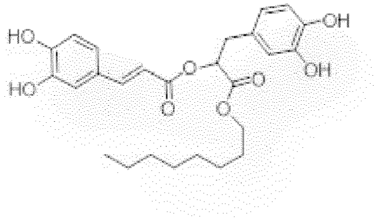

Les inventeurs ont constaté qu'un mélange particulier de ces différents extraits présentait une synergie particulière ce qui permettait d'obtenir, de manière inattendue, des propriétés antioxydante encore plus élevées comme le démontrent les résultats obtenus à l'exemple 4.

Dans un mode de réalisation préférée, l'extrait (II) est obtenu par extraction alcoolique au moyen d'un alcool monohydrique et/ou d'un glycol, éventuellement mélangé avec de l'eau.

De manière particulièrement avantageuse, la composition comprend un mélange des extraits (I), (II) et (III), où le rapport en poids des extraits (!!)/(!!!) est compris entre 0,5 et 1,5 et le rapport en poids des extraits (I)/(II) est compris entre 2 et 4.

De manière encore plus avantageuse, la composition comprend un mélange des extraits (I), (II) et (III), où le rapport en poids des extraits (I)/(II)/(III) est 3/1/1.

Les inventeurs ont observé, de manière particulièrement inattendue, que ces proportions particulières présentaient un effet antioxydant accru, comme le démontre les résultats du tableau 3 à l'exemple 4. Par ailleurs, en fonction de la polarité chacune des molécules présentes, lesdits extraits auront une action donnée dans les différents compartiments cellulaires et traverseront plus aisément les barrières formées par les différentes couches du derme et de l'épiderme.

Avantageusement, la composition est adaptée à une application par voie topique.

Dans un mode particulier de réalisation de la composition, ledit extrait (I) ou le mélange des extraits (I), (II) et (III) représente une teneur allant de 0,01 à 10% en poids en particulier à raison de 0,1 à 10%, de préférence de 1 à 5% en poids total de la composition.

Les exemples de compositions cosmétiques et dermatologiques testés à l'exemple 5 ont démontré que ces concentrations permettaient de conserver une activité antioxydante remarquablement élevée une fois le principe actif introduit dans une composition adaptée à une application par voie topique.

La composition cosmétique ou dermatologique selon l'invention comprend ainsi un extrait obtenu selon un procédé d'extraction de tiges de menthe poivrée comprenant au moins les étapes telles que:
1. prendre des tiges de menthe poivrée préférentiellement séchées,
2. broyer lesdites tiges,
3. extraire lesdites tiges avec un mélange de solvants hydro-alcooliques composé de préférence d'un mélange éthanol/eau à une température supérieure à 50°C,
4. tamiser afin d'éliminer les résidus végétaux,
5. préférentiellement décolorer par contact avec du charbon activé pendant une heure, puis séparer le mélange du résidu de carbone à l'aide d'une microfiltration pour obtenir un extrait,
6. éliminer le solvant alcoolique de préférence par évaporation et concentrer l'extrait,
7. acidifier avec de l'acide sulfurique pour atteindre un pH<3 et ajouter un solvant organique tel que de l'acétate d'éthyle,
8. séparer l'extrait contenu dans la phase organique de la phase aqueuse, éliminer la phase aqueuse puis éliminer le solvant organique pour obtenir une poudre, et
9. une étape de fonctionnalisation par addition 1,3-propanediol, ou de glycerol, ou l'alcool octylique.

Dans une première variante de l'invention, la composition comprend un extrait de menthe poivrée (II) obtenu selon un procédé d'extraction hydro-alcoolique de tiges de menthe poivrée comprenant les étapes suivantes:
1) -prendre des tiges de menthe poivrée préférentiellement séchées,
2) -broyer lesdites tiges, typiquement à une finesse inférieure à 2 cm,
3) -extraire préférentiellement deux fois lesdites tiges avec un mélange de solvants hydro-alcooliques typiquement composé de 55% d'éthanol à 96° et 45% d'eau (v/v) dans des conditions de 60° C et pendant 2 heures minimum,
4) -tamiser préférentiellement jusqu'à 5µm afin d'éliminer les résidus végétaux,
5) -préférentiellement décolorer par contact avec du charbon activé pendant une heure afin d'éliminer les pigments tels que les chlorophylles et xanthophylles puis séparer le mélange du résidu de carbone à l'aide d'une microfiltration (jusqu'à 1µm) pour obtenir un extrait,
6) -éliminer le solvant alcoolique de préférence par évaporation et concentrer l'extrait,
7) -acidifier typiquement avec de l'acide sulfurique pour atteindre un pH<3 et ajouter un solvant organique typiquement de l'acétate d'éthyle pour effectuer une purification liquide / liquide,
8) -séparer l'extrait contenu dans la phase organique de la phase aqueuse, éliminer la phase aqueuse puis éliminer le solvant organique par exemple sous vide pour obtenir une poudre,
9) -préférentiellement, ajouter du 1,3-propanediol à ladite poudre.

Dans une seconde variante de l'invention, la composition comprend un extrait de menthe poivrée (I) obtenu selon un procédé comprenant les étapes suivantes:
1) prendre l'extrait (II) obtenu précédemment
2) -ajouter du glycérol à ladite poudre,
3) -effectuer l'estérification complète de l'acide rosmarinique et des dérivés préférentiellement en ajoutant une résine échangeuse d'ions au mélange précédent et pendant 7 jours minimum à 70° C, puis, éliminant la résine échangeuse d'ions par tamisage,
4) -de préférence, décolorer par contact avec du charbon activé pendant une heure puis éliminer le résidu de carbone par filtration (jusqu'à 4µm),
5) -ajouter du glycérol et de préférence une solution aqueuse.

Dans une troisième variante de l'invention, la composition comprend un extrait de menthe poivrée (III) obtenu selon un procédé comprenant les étapes suivantes:
1) prendre l'extrait (II) obtenu précédemment
2) -ajouter de l'alcool octylique à ladite poudre,
3) -effectuer l'estérification complète de l'acide rosmarinique en ajoutant une résine échangeuse d'ions au mélange précédent pendant 7 jours à 70° C, puis, en éliminant la résine échangeuse d'ions par tamisage,
4) -de préférence, décolorer par contact avec du charbon activé pendant une heure et éliminer ensuite le résidu de carbone par filtration (jusqu'à 4µm),
5) -préférentiellement, éliminer l'alcool octylique résiduel par évaporation et ajouter du dipropylène glycol.

Les inventeurs ont observé que les extraits obtenus selon ces différentes étapes étaient particulièrement riches en composés d'intérêt et permettait leurs utilisations au sein de compositions cosmétique ou dermatologique. Les composés de formules (I), (II) et (III) précédemment évoqués sont donc préférentiellement obtenus par ces procédés.

L'invention concerne également l'utilisation d'un extrait de menthe poivrée (I) tel que décrit précédemment, en tant qu'agent antioxydant dans une composition cosmétique ou dermatologique.

De manière avantageuse, la composition selon l'invention comprend également un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique comprend également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre un additif usuel dans le domaine, tel que par exemple un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir:
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglyceride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycerides), les huiles végétales, le phytosqualane, les alcanes (undecane, tridecane)
   Ledit agent émollient sera présent dans la composition à une teneur de l'ordre de 0 à 30%, de préférence 0.5 à 10% en poids total de la composition.
- Un ou plusieurs agent(s) humectants, tels que les polyols (glycérine, le propylène glycol, le propanediol), les sucres, les glycosaminoglycanes tels que l'acide hyaluronique et ses sels et esters; et les polyquaterniums tel que le lipidure PMB
   Ledit agent humectant sera présent dans la composition à une teneur de l'ordre de 0 à 30%, de préférence 0.005 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP)
   Ledit agent gélifiant et/ou épaississant sera présent dans la composition à une teneur de l'ordre de 0 à 10% en poids total de la composition.
- Un ou plusieurs agent(s) tensioactif(s), tels que les lecithines, les dérivés de sucre (les dérivés de glucosides ou de xylosides commercialisés sous le nom MONTANOV 68, MONTANOV 202, Montanov 82, MONTANOV L, EASYNOV), les phosphates (C20-22 alkyl phosphate commercialisé sous le nom SENSANOV WR) présent dans une teneur de l'ordre de 0 à 8%, de préférence 0,5 à 3% en poids total de la composition.
- Un ou plusieurs corps gras liquide(s) à température ambiante, communément dénommé(s) huiles(s), volatile(s) ou non volatile(s), hydrocabroné(s), siliconé(s), linéaire(s), cyclique(s) ou ramifié(s), par exemple, l'isododécane, le cyclopentadimethylsiloxane, les diméthicone, l'isononanoate d'isononyle, le pentaerythrityl tetraisostéarate, etc., de préférence à raison de 0 à environ 10%, de préférence 0,5 à 5% en poids total de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, les oligo éléments, l'allantoïne, l'adenosine, les peptides (Palmitoyl Tripeptide-1, Palmitoyl Tetrapeptide-7, Palmitoyl Pentapeptide-4, Acetyl Dipeptide-1 Cetyl Ester, Acetyl Tetrapeptide-5 commercialisés sous le nom MATRIXYL 3000, N-Palmitoyl-Rigin, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, les beta hydroxyacides tel que l'acide salicylique et ses dérivés etc.
   Ledit agent actif sera présent dans la composition à une teneur de l'ordre de 0 à 10% en poids total de la composition.
- Une ou plusieurs poudres tels que, par exemple, la silice, le nylon-12, la cellulose, le boron nitride, les Silicones élastomères (Dimethicone/vinyl dimethicone crosspolymers and dimethicone crosspolymers commercialisés sous le nom DC 9040, DC 9041, DC 9045, KSG-15, KSG-16, KSG-016F, KSG-18, lauryl dimethicone/vinyl dimethicone crosspolymers commercialisés sous le nom KSG-31, KSG-32, KSG-41, KSG-42, KSG-43, KSG-44) de préférence à raison de 0 à environ 10% en poids total de la composition.

D'autres additifs habituellement utilisés en cosmétique peuvent également être présents dans la composition selon l'invention, notamment des conservateurs (phenoxyethanol, pentylene glycol, caprylyl glycol, chlorphenesin...), des agents antioxydants ou des parfums bien connus dans le domaine technique.

L'homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

L'invention est illustrée de manière non limitative par les exemples ci-dessous.

On distingue trois extraits différents appelés ci-après; un extrait de menthe poivrée (II), un extrait de menthe poivrée (I) et un extrait de menthe poivrée (III).

### Exemple 1 :

Préparation d'un extrait de menthe poivrée (II) à partir de l'extraction hydro-alcoolique des tiges séchées et de la purification à l'acétate d'éthyle, selon les étapes suivantes:
1) Les tiges séchées de menthe poivrée sont broyées à une finesse inférieure à 2 cm,
2) Les tiges sont ensuite extraites deux fois avec un mélange de solvants composé de 55% d'éthanol à 96 ° et 45% d'eau (v/v) dans des conditions de 60 ° C et 2 heures minimum,
3) Le mélange est tamisé (jusqu'à 5µm) afin d'éliminer les résidus végétaux,
4) Le mélange résultant est soumis à une décoloration par contact avec du charbon activé pendant une heure afin d'éliminer les pigments tels que les chlorophylles et xanthophylles
5) Le mélange décoloré est séparé du résidu de carbone à l'aide d'une microfiltration (jusqu'à 1µm),
6) L'éthanol du mélange est éliminé par évaporation et on concentre l'extrait,
7) L'extrait concentré est acidifié avec de l'acide sulfurique pour atteindre un pH de <3 et de l'acétate d'éthyle est ajouté pour effectuer une purification liquide / liquide,
8) La phase d'acétate d'éthyle supérieure est séparée de la phase aqueuse inférieure. Le solvant acétate d'éthyle de la phase supérieure est éliminé sous vide pour obtenir une poudre,
9) 1, 3-propanediol est ajouté à la poudre. Pour faciliter la solubilisation, l'éthanol peut être ajouté et éliminé par évaporation.

La menthe poivrée (II) résultant est composé de 5% d'extrait sec (p/p) et 95% de 1, 3-propanediol. Cet extrait est principalement composé d'acide rosmarinique à une teneur comprise entre 1.5-2%.

### Exemple 2 :

Préparation d'un extrait de menthe hydrophilisée (I) () à partir de l'extraction hydro-alcoolique de tiges séchées, purifiée à l'acétate d'éthyle et estérifiée avec du glycérol, selon les étapes suivantes:
1) Mêmes étapes que menthe poivrée (II) de 1) à 8)
2) La poudre est ajoutée à la glycérine,
3) Une résine échangeuse d'ions tel qu'une résine échangeuse de cations est ajouté au mélange précédent et l'estérification complète de l'acide rosmarinique et des dérivés est effectuée pendant 7 jours minimum à 70 ° C. Ensuite, la résine échangeuse d'ions est éliminée par tamisage,
4) Le mélange résultant est soumis à une décoloration par contact avec du charbon activé pendant une heure. Le résidu de carbone est ensuite éliminé par filtration (jusqu'à 4µm),
5) Enfin, l'eau et le glycérol sont ajoutés pour obtenir la menthe poivrée Hyros composée de 5% d'extrait sec (p/p), 67% de glycerol et 28% d'eau.

Cet extrait (I) est principalement composé de glycéryle rosmarinate à une teneur comprise entre 1-1.7%.

### Exemple 3 :

Préparation d'un extrait de menthe Lipophilisée (III) () à partir de l'extraction hydro-alcoolique de tiges séchées, purifiée à l'acétate d'éthyle et estérifiée avec de l'alcool octylique, selon les étapes suivantes:
1) Mêmes étapes que menthe poivrée (II) de 1) à 8)
2) La poudre est ajoutée à l'alcool octylique,
3) Une résine échangeuse d'ions tel qu'une résine échangeuse de cations est ajouté au mélange précédent et l'estérification complète de l'acide rosmarinique et des dérivés est effectuée pendant 7 jours minimum à 70 ° C. Ensuite, la résine échangeuse d'ions est éliminée par tamisage,
4) Le mélange résultant est soumis à une décoloration par contact avec du charbon activé pendant une heure. Le résidu de carbone est ensuite éliminé par filtration (jusqu'à 4µm),
5) L'alcool octylique résiduel est éliminé par évaporation et, enfin, le dipropylène glycol est ajouté pour obtenir la menthe poivrée Liros composé de 5% d'extrait sec (p/p), 95% de dipropylèneglycol.

Cet extrait (III) est principalement composé d'octyle rosmarinate à une teneur comprise entre 1.5-2.7%.

La matière première mise en œuvre consiste, par exemple, en des tiges de menthe poivrées d'une espèce de *Mentha Piperita,* que l'on peut broyer ou réduire en morceaux de manière usuelle.

Les tiges *Mentha piperita* utilisées pour ces extractions contiennent de l'acide rosmarinique mais également des dérivés.

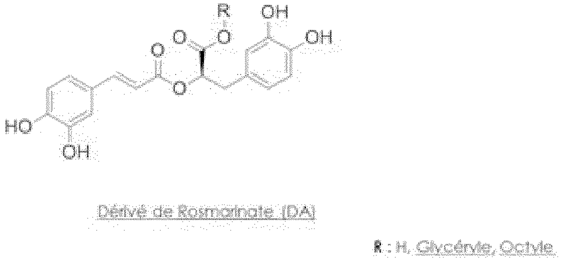

### Exemple 4 : Mesure de l'activité antioxidante des extraits et de leurs mélanges

L'activité antioxydante totale est l'aptitude d'un composé ou d'un ensemble de composés antioxydants à piéger les radicaux libres. Elle a été évaluée à l'aide de la PAOT Technologie® développée par l'Institut Européen des Antioxydants. Cette méthode donne le Pouvoir Antioxydant et Oxydant Total (PAOT) de la matrice analysée.

La mesure se fait en milieu liquide, par solubilisation du produit dans un milieu réactionnel, puis dosage direct de son activité en solution.

Le milieu réactionnel de la technologie PAOT est composé principalement de solutions physiologiques avec un pH 6,7 à 7,2 (pour simuler les conditions biologiques). 20µl d'échantillon sont mélangés à 1 ml du milieu réactionnel. Des électrodes spécifiques sont plongées dans la solution pendant maximum 7 minutes à une température comprise entre 24-27°C. Le principe est basé sur le changement dans le rapport des formes oxydées et réduites des composants du milieu.

Ce changement résulte de la variation des concentrations des formes oxydées/réduites durant la réaction (1) pour les antioxydants et la réaction (2) pour oxydants :
- Milieu réactionnel +AO (Antioxydant) => Milieu réactionnel+ AOOx (Résultat d'oxydation de l'antioxydant) (1)
- Milieu réactionnel + OA (Oxydant) => Milieu réactionnel + OA Red (Résultat de la réduction de l'oxydant) (2)

Les résultats peuvent être exprimés en unités PAOTScore® (Pouvoir AntiOxydant Total) ou POTScore® (Pouvoir Oxydant Total) par litre ou par gramme de produit analysé (PAOT Score/I ou POT Score®/g de produit). Il peut être également peut être exprimé en µM/l ou µM/g de molécules de référence (antioxydant et oxydant).

Les conditions expérimentales sont été réalisées en triplicata (n=3)
Définition des extraits et de leurs mélanges :
- MEL 1: 100% de Menthe poivrée (I)
- MEL 2 : 100% de Menthe poivrée (II)
- MEL 3 : 100% de Menthe poivrée (III)
- MEL 4 : 66.6% de Menthe poivrée (I), 16.6% de Menthe poivrée (II) et 16.6 % de Menthe poivrée (III)

Les résultats sont rapportés dans le tableau ci-dessous :

**Tableau 1 :**

| | PAOT score |
|---|---|
| MEL 1 | 528 |
| MEL 2 | 481 |
| MEL 3 | 353 |
| MEL 4 | 571 |

L'activité antioxydante totale est exprimée en équivalent référence. L'équivalence est exprimée en gramme de référence par litre de produit. Plus la valeur de l'équivalence est élevée, plus l'efficacité produit est élevée.

Pour être considéré comme ayant des propriétés antioxydantes très efficaces, il faut que le PAOT-Score soit > à 50. Entre 40 et 49 PAOT-Score, l'activité antioxydante est considérée comme efficace et pour un PAOT-Score entre 25 et 39, l'activité antioxidante est évaluée comme moyenne.

Tous les échantillons dérivés à partir des extraits obtenus dans les exemples 1, 2 et 3 montrent donc des propriétés antioxydantes particulièrement remarquables.

Des tests de contrôle sont effectués pour mesurer l'activité antioxydante des solvants E15/177 ZEMA, E15/178 DPG (Dilution 1/2) et E15/179 Glycérol/Eau, leur score PAOT reste inférieur à 3 ce qui montre leur absence de rôle de l'activité antioxydante observé dans les échantillons ci-dessus.

On constate que l'échantillon MEL 1, l'extrait de menthe poivrée (I) présente le meilleur score de pouvoir antioxydant, et que le MEL 4, le mélange des 3 extraits dans le ratio 3/1/1 présente une synergie inattendue.

### Exemple 5:

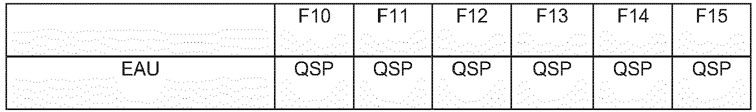

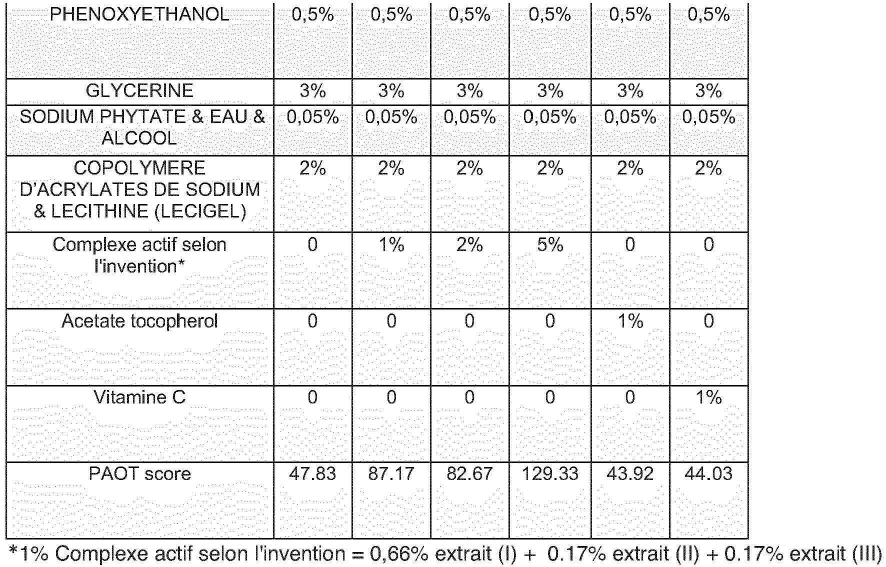

Le score PAOT a également été évalué au sein de plusieurs compositions cosmétiques de type sérum à différentes concentrations. Les compositions ayant données les meilleurs résultats ont été obtenus pour celles contenant entre 1 et 5% en poids de complexe actif par rapport au poids total et plus particulièrement pour la composition comprenant 5% en poids de complexe actif, dans cette dernière le pouvoir antioxydant mesuré a donné un score PAOT d'environ 130. Cet exemple de composition cosmétique peut donc être considéré comme ayant des propriétés antioxydantes très efficaces.

On constate également qu'à concentration équivalente (1%), la formule avec le complexe actif révèle un score 2 fois plus haut par rapport aux formules avec les témoins Tocophérol et Vitamine C (leurs deux scores étant équivalents).

## Revendications

1. Extrait de menthe poivrée (I) obtenu par extraction des tiges avec un mélange de solvants hydro-alcooliques de préférence d'un mélange éthanol/eau et estérification avec du glycérol, ledit extrait de menthe poivrée (I)comprenant au moins un composé de formule (I) :

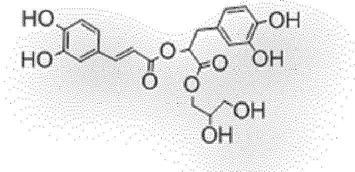

2. Composition cosmétique ou dermatologique, **caractérisée en ce qu'**elle comprend, dans un milieu physiologiquement acceptable, un extrait de menthe poivrée (I) selon la revendication 1.

3. Composition selon la revendication 2, **caractérisée par le fait qu'**elle comprend également un extrait de menthe poivrée (II) obtenu par extraction des tiges avec un mélange de solvants hydro-alcooliques, de préférence d'un mélange éthanol/eau, comprenant au moins un composé de formule (II) : 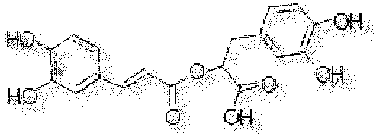 et un extrait de menthe poivrée (III) obtenu par extraction des tiges de menthe poivrée avec un mélange de solvants hydro-alcooliques, de préférence avec un mélange éthanol/eau et estérification avec de l'alcool octylique, ledit extrait de menthe poivrée (III) comprenant au moins un composé de formule (III) :

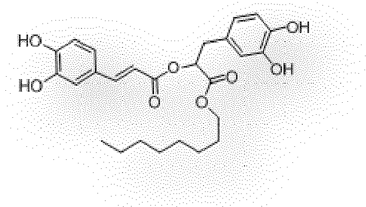

4. Composition selon la revendication 3, **caractérisée en ce que** l'extrait hydro-alcoolique (II) est obtenu par extraction alcoolique au moyen d'un alcool monohydrique et/ou d'un glycol, éventuellement mélangé avec de l'eau.

5. Composition selon l'une des revendications 3 ou 4, **caractérisée par le fait qu'**elle comprend, un mélange des extraits (I), (II) et (III), où le rapport en poids des extraits (II)/(III) est compris entre 0,5 et 1,5 et le rapport en poids des extraits (I)/(II) est compris entre 2 et 4.

6. Composition selon la revendication précédente, **caractérisée en ce que** le rapport en poids des extraits (I)/(II)/(III) est 3/1/1.

7. Composition selon l'une quelconque des revendications précédentes, **caractérisée en ce qu'**elle est adaptée à une application par voie topique.

8. Composition selon l'une quelconque des revendications précédentes, **caractérisée par le fait que** ledit extrait (I) ou le mélange des extraits (I), (II) et (III) représente une teneur allant de 0,01 à 10% en poids en particulier à raison de 0,1 à 10%, de préférence de 1 à 5% en poids total de la composition.

9. Utilisation cosmétique de la composition selon l'une quelconque des revendications 1 à 8 pour réduire la présence de radicaux libres impliqués dans les phénomènes de vieillissement cellulaire de la peau.

10. Utilisation d'un extrait de menthe poivrée (I) selon la revendication 1 en tant qu'agent antioxydant dans une composition cosmétique ou dermatologique.

## Patentansprüche

1. Pfefferminzextrakt (I), der durch Extrahieren der Stängel mit einer Mischung aus hydroalkoholischen Lösungsmitteln, bevorzugt einer Ethanol/Wasser-Mischung, und Verestern mit Glycerin, erhalten wird, wobei der Pfefferminzextrakt (I) wenigstens eine Verbindung der Formel (I) umfasst:

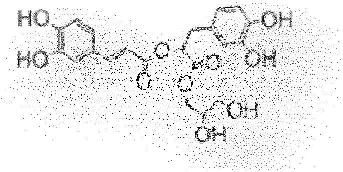

2. Kosmetische oder dermatologische Zusammensetzung, **dadurch gekennzeichnet, dass** sie in einem physiologisch akzeptablen Medium einen Pfefferminzextrakt (I) nach Anspruch 1 umfasst.

3. Zusammensetzung nach Anspruch 2, **dadurch gekennzeichnet, dass** sie auch einen Pfefferminzextrakt (II) umfasst, der durch Extrahieren der Stängel mit einer Mischung aus hydroalkoholischen Lösungsmitteln, bevorzugt einer Ethanol/Wasser-Mischung, erhalten wird, die wenigstens eine Verbindung der Formel (II) umfasst: 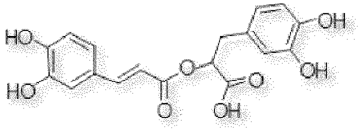 und einen Pfefferminzextrakt (III), der durch Extrahieren der Pfefferminzstängel mit einer Mischung aus hydroalkoholischen Lösungsmitteln, bevorzugt einer Ethanol/Wasser-Mischung, und Verestern mit Octylalkohol erhalten wird, wobei der Pfefferminzextrakt (III) wenigstens eine Verbindung der Formel (III) umfasst:

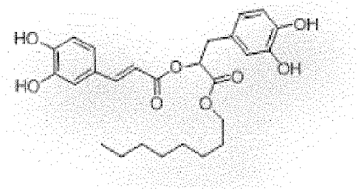

4. Zusammensetzung nach Anspruch 3, **dadurch gekennzeichnet, dass** der hydroalkoholische Extrakt (II) durch alkoholische Extraktion mittels eines einwertigen Alkohols und/oder eines Glykols, gegebenenfalls gemischt mit Wasser, erhalten wird.

5. Zusammensetzung nach einem der Ansprüche 3 oder 4, **dadurch gekennzeichnet, dass** diese eine Mischung der Extrakte (I), (II) und (III) umfasst, wobei das Gewichtsverhältnis der Extrakte (II)/(III) zwischen 0,5 und 1,5 und das Gewichtsverhältnis der Extrakte (I)/(II) zwischen 2 und 4 liegt.

6. Zusammensetzung nach dem vorhergehenden Anspruch, **dadurch gekennzeichnet, dass** das Gewichtsverhältnis der Extrakte (I)/(II)/(III) 3/1/1 beträgt.

7. Zusammensetzung nach einem der vorhergehenden Ansprüche, **dadurch gekennzeichnet, dass** sie für eine topische Anwendung geeignet ist.

8. Zusammensetzung nach einem der vorstehenden Ansprüche, **dadurch gekennzeichnet, dass** der Extrakt (I) oder die Mischung der Extrakte (I), (II) und (III) einen Gehalt im Bereich von 0,01 bis 10 Gew.-%, insbesondere von 0,1 bis 10 %, bevorzugt von 1 bis 5 % des Gesamtgewichts der Zusammensetzung darstellt.

9. Kosmetische Verwendung der Zusammensetzung nach einem der Ansprüche 1 bis 8 zur Reduzierung des Vorhandenseins freier Radikale, die an den zellulären Alterungserscheinungen der Haut beteiligt sind.

10. Verwendung eines Pfefferminzextrakts (I) nach Anspruch 1 als Antioxidans in einer kosmetischen oder dermatologischen Zusammensetzung.

## Claims

1. Peppermint extract (I) obtained by the extraction of peppermint stems with a mixture of hydroalcoholic solvents, preferably an ethanol/water mixture, and esterification with glycerol, said peppermint extract (I) comprising at least a compound of formula (I):

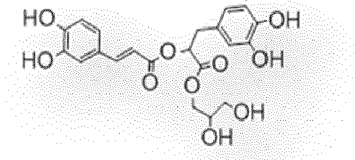

2. Cosmetic or dermatological composition, **characterised in that** it comprises, in a physiologically acceptable medium, a peppermint extract (I) according to claim 1.

3. Composition according to claim 2, **characterised by** the fact that it also comprises a peppermint extract (II) obtained by the extraction of stems with a mixture of hydroalcoholic solvents, preferably an ethanol/water mixture, comprising at least a compound of formula (II): 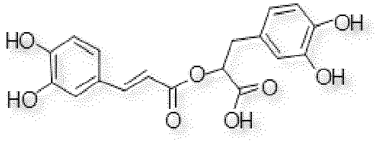 and a peppermint extract (III) obtained by the extraction of peppermint stems with a mixture of hydroalcoholic solvents, preferably with an ethanol/water mixture, and esterification with octyl alcohol, said peppermint extract (III) comprising at least one compound of formula (III):

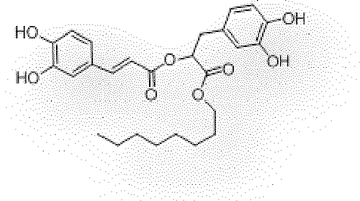

4. Composition according to claim 3, **characterised in that** the hydroalcoholic extract (II) is obtained by alcohol extraction by means of a monohydric alcohol and/or a glycol, optionally mixed with water.

5. Composition according to either claim 3 or 4, **characterised by** the fact that it comprises a mixture of extracts (I), (II) and (III), wherein the ratio by weight of the extracts (II)/(III) is between 0.5 and 1.5 and the ratio by weight of the extracts (I)/(II) is between 2 and 4.

6. Composition according to the preceding claim, **characterised in that** the ratio by weight of the extracts (I)/(II)/(III) is 3/1/1.

7. Composition according to any of the preceding claims, **characterised in that** it is suited to topical application.

8. Composition according to any of the preceding claims, **characterised by** the fact that said extract (I) or the mixture of extracts (I), (II) and optionally (III) represents a content ranging from 0.01% to 10% by weight, in particular from 0.1% to 10%, preferably from 1% to 5% by weight, with respect to the total weight of the composition.

9. Cosmetic use of a composition according to any of claims 1 to 8 to reduce the presence of free radicals involved in the phenomena of cell ageing of the skin.

10. Use of a peppermint extract (I) according to claim 1 as an antioxidant in a cosmetic or dermatological composition.
